# EUROPEAN PATENT SPECIFICATION

(11) **EP 1 719 517 B1**
(45) Date of publication and mention of the grant of the patent: **25.08.2010**
(21) Application number: 05710509.0
(22) Date of filing: 22.02.2005
(51) Int. Cl.: A61K 33/14, A61P 7/08, A61P 27/04

(54) **ARTIFICIAL PHYSIOLOGICAL SALT SOLUTION AND PROCESS FOR PRODUCING THE SAME**
KÜNSTLICHE PHYSIOLOGISCHE SALZLÖSUNG UND VERFAHREN ZU IHRER HERSTELLUNG
SOLUTION SALINE PHYSIOLOGIQUE ARTIFICIELLE ET PROCEDE SERVANT A PRODUIRE CELLE-CI

(30) Priority: 27.02.2004 JP 2004053720
(43) Date of publication of application: 08.11.2006
(73) Proprietor: Nihon Trim Co., Ltd., Osaka-shi, Osaka 5310076 (JP)
(72) Inventor: KABAYAMA, Shigeru, Nihon Trim Co., Ltd., Osaka-shi, Osaka 5310076 (JP); MORISAWA, Shinkatsu, Nihon Trim Co., Ltd., Osaka-shi, Osaka 5310076 (JP)
(74) Representative: Lipscombe, Martin John
(86) International application number: PCT/JP2005/002778
(87) International publication number: WO 2005/082384

(56) References cited:
- EP-A- 0 698 388
- EP-A- 0 826 636
- EP-A- 1 101 510
- WO-A-00/76475
- JP-A- 1 151 462
- JP-A- 8 169 835
- JP-A- 9 500 380
- JP-A- 10 118 653
- JP-A- 2000 157 977
- JP-A- 2001 145 880
- JP-A- 2002 254 078
- US-A- 5 405 742
- US-A1- 2003 186 452

## Description

### Technical Field

The present invention relates to an artificial physiological salt solution and a manufacturing method for the same.

### Background Art

In recent years, the number of people who are suffering from symptoms such as dry eyes, including those who use contact lenses, strange or uncomfortable feelings due to a foreign body, tired eyes, itchiness of the eye, an increase in blinking, bloodshot eyes and the like has been increasing steadily. Having dry eyes is a condition where the amount of tears, which cover the surface of the eyes and function to expel pathogens from the environment and bring nutrition to the diaphragm, decreases due to a multitude of reasons, and thereby, the function of the tears, which cover the surface of the eyes, is hindered. As a result of this, symptoms such as those described above appear: dry eyes, strange or uncomfortable feelings due to a foreign body, tired eyes, itchiness of the eye, an increase in blinking, bloodshot eyes and the like. As for the cause of dry eyes, a reduction in the secretion function of lachrymal glands due to aging, conjunctivitis, vitamin A deficiency, Sjogren's syndrome, Steven-Johnson syndrome, rheumatism, diabetes, as a side effect of medicine and the like can be cited, and therefore, hyposecretion type dry eyes are caused. In addition, in recent years, excessive evaporation type dry eyes are caused by the excessive lachrymal evaporation as a result of wearing contact lenses, a reduction in the number of blinks while doing work on VDT (Visual Display Terminals) and the like.

As for a conventional remedy for dry eyes, methods for giving advice on one's lifestyle concerning the humidification of a room and regular blinking, methods for applying artificial lachrymal eye drops frequently, methods for suppressing the amount of evaporation of lachrymal fluid by wearing an eye shield and methods for closing lachrymal puncta have been used with a basic concept of maintaining balance between the amount of secretion and the amount of evaporation of lachrymal fluid. In recent years, it has already been clarified that an oxidation reaction or an inflammatory reaction is recognized in the lachrymal fluid layer of patients suffering from dry eyes (see, for example, Albert J. Augustin et al., "Oxidative Reactions in the Tear Fluid of Patients Suffering from Dry Eyes," Graefe's Archive for Clinical and Experimental Ophthalmology, Springer, vol. 233, no. 11, 1995, pages 694-698 (Non-patent Document 1)). Therefore, attention has been paid to the change in inflammation which causes or which is a result from dry eyes, and it has been attempted to alleviate the symptoms of dry eyes by applying eye drops with steroids (see, for example, Avni M. Avunduk et al., "The Comparison of Efficacies of Topical Corticosteriods and Nonsteroidal Anti-inflammatory Drops on Dry Eye Patients: A Clinical and Immunocytochemical Study," American Journal of Ophthalmology, Elsevier Science, vol. 136, no. 4, October 2003, pages 593-602 (Non-patent Document 2)).

However, it is difficult in a modem society, where one cannot help but work busily, a remedy method with a basic concept of maintaining the balance between the amount of secretion and the amount of the evaporation of lachrymal fluid is sufficiently carried out, even in the case where advice is given on one's lifestyle, and thus, effects cannot essentially be expected. In addition, as for the eye drops with steroids, though the effects thereof are expected to be assured, a reduction in the effects as a result of frequent usage and critical side effects such as rebound at the time when usage is stopped can also surely be expected. Accordingly, the development of a novel composition, which is applicable in the cleaning of eyes or in the eye drops and can suppress an oxidation reaction and an inflammatory reaction without the occurrence of side effects, has been desired.

In addition, conventional cleaning solutions which are used to clean the nasal cavity for the purpose of alleviating allergy symptoms such as hay fever have been known. As for the cleaning solution for the nasal cavity, a conventional physiological salt solution or the like is used and has no ability of suppressing an oxidation reaction or an inflammatory reaction. Therefore, in the same manner as in the above described case of a cleaning solution for the eyes, the development of a cleaning solution for the nasal cavity which can sufficiently suppress an oxidation reaction and an inflammatory reaction is desired at present.

In recent years, artificial infusions such as total amino acid infusions, a sugar/electrolyte/amino acid liquid for high calorie infusions, electrolyte infusions, a sugar/electrolyte liquid for high calorie infusions, intraperitoneal dialysate and dialysate for an artificial kidney are frequently used, for example, in a medical site. Such artificial infusions are used for the purpose of recovery after an operation, replenishing from dehydration, the maintenance of life and the like, and in such conditions, the body is internally damaged, and it is possible that the damage is a result of oxidation caused by active oxygen. In addition, it is possible that a problem of damage through oxidation caused by active oxygen in the same manner arises with artificial amniotic fluid which are used for maintaining the life of a fetus at the site of giving birth, and protective solution such as protective solution for myocardia, which is used at the time of heart operations, in addition to artificial infusions. Accordingly, though it is desired for the artificial infusions, artificial amniotic fluid and protective solutions to be able to suppress oxidation reactions and inflammatory reactions within a human body, no artificial infusions, artificial amniotic fluid or protective solutions, in which the protective substance against damage through oxidation has been incorporated, has yet been developed.

In addition, no cell/tissue culture solution which is prepared to have a composition that is close to the body fluids of a human body so as not to negatively affect the cells or the tissue that is cultured, and contains a protective substance that can sufficiently prevent damage through oxidation has not yet existed up to current times. Cells and tissue which is cultured using a cell/tissue culture solution is artificially in a state of being easily handled, unlike the cells/tissue within a human body, and therefore, are susceptible to stress through oxidation more easily in comparison with the cells/tissue within a human body, and thus, it can be said that the development of a novel cell/tissue culture solution which can prevent such damage through oxidation is particularly useful.

Non-patent Document 1: Albert J. Augustin et al., "Oxidative Reactions in the Tear Fluid of Patients Suffering from Dry Eyes," Graefe's Archive for Clinical and Experimental Ophthalmology, Springer, vol. 233, no. 11, 1995, pages 694-698

Non-patent Document 2: Avni M. Avunduk et al., "The Comparison of Efficacies of Topical Corticosteriods and Nonsteroidal Anti-inflammatory Drops on Dry Eye Patients: A Clinical and Immunocytochemical Study," American Journal of Ophthalmology, Elsevier Science, vol. 136, no. 4, October 2003, pages 593-602

### Disclosure of the Invention

### Problems to be Solved by the Invention

The present invention is provided in order to solve the above described problems, and an object thereof is to provide a novel artificial physiological salt solution, which has active oxygen eliminating activity and anti-inflammation effects and can be used in a multitude of applications such as organ cleaning solutions (cleaning solutions for the eyes, cleaning solutions for the nasal cavity and the like), artificial infusions, artificial amniotic fluid, protective solutions, and cell/tissue culture solutions, as well as to provide a manufacturing method for the same.

### Means for Solving the Problems

An artificial physiological salt solution according to the present invention is characterized in that the active hydrogen reaction value is 0.01 to 1, the pH is 6.0 to 7.9 and the osmotic pressure is 260 mOsm/L to 320 mOsm/L.

Here, it is preferable for the artificial physiological salt solution according to the present invention to include sodium ions, potassium ions and chloride ions.

It is preferable for the artificial physiological salt solution according to the present invention to include not higher than 200 mEq/L of sodium ions.

It is preferable for the artificial physiological salt solution according to the present invention to include not higher than 100 mEq/L of potassium ions.

It is preferable for the artificial physiological salt solution according to the present invention to include not higher than 200 mEq/L of chloride ions.

In addition, it is preferable for the artificial physiological salt solution according to the present invention to have electrolytic reduction water in which an adjustment of the ion balance has been carried out.

Furthermore, it is preferable for the oxidation-reduction potential in the artificial physiological salt solution according to the present invention to be -800 mV to +200 mV.

The artificial physiological salt solution according to the present invention are appropriate for use in the following applications (a) to (c).
(a) Organ cleaning solutions
(b) Artificial infusions, artificial amniotic fluid or protective solutions
(c) Cell/tissue culture solutions

The present invention also provides a manufacturing method for an artificial physiological salt solution which is characterized in that electrolytic reduction water is adjusted in such a manner that the active hydrogen reaction value is 0.01 to 1, the pH is 6.0 to 7.9 and the osmotic pressure is 260 mOsm/L to 320 mOsm/L.

It is preferable for the manufacturing method for an artificial physiological salt solution according to the present invention to further include the step of adjusting the ion balance in electrolytic reduction water by adding sodium chloride and/or potassium chloride.

### Effects of the Invention

The artificial physiological salt solution according to the present invention exhibits active oxygen eliminating activity and the function of suppressing chemotactic activity and migratory activity in polymorphonuclear leucocytes, in particular, neutrophils, which are inflammatory cells, and furthermore, do not negatively affect the organs, the tissue and the cells to which it is applied, and therefore, can be appropriately used in a multitude of applications as an artificially prepared physiological salt solution.

The artificial physiological salt solution according to the present invention, for example, has strong active oxygen eliminating activity and anti-inflammation effects in comparison with conventional cleaning solutions for the eyes other than those with steroids, and therefore, in the case where it is applied to cleaning solutions for the eyes, sufficient effects can be gained with a smaller number of times of cleaning or application of eye drops than that according to the prior art. Accordingly, even in a modem society where one cannot help but work busily, the essential effects can be expected in the care of eyes. In addition, the artificial physiological salt solution according to the present invention can prevent inflammation of the eyes and can prevent the tissue of the eyes from being damaged by active oxygen or a substance that brings about active oxygen without causing undesired side effects, unlike the case where a conventional solution having steroids is used, and thereby, effects can be gained where symptoms such as dry eyes/strange or uncomfortable feelings due to a foreign body, tired eyes, itchiness of the eye, an increase in blinking, bloodshot eyes and the like can be alleviated.

In addition, in the case where the artificial physiological salt solution according to the present invention is applied as a cleaning solution for the nasal cavity, effects can be gained, such that dust, sundry germs, pollen and the like within the nasal cavity can be removed, and the inside of the nasal cavity can be appropriately cleaned, and in addition, allergic symptoms such as hay fever can be suppressed without causing undesired side effects, due to the antioxidation effects and anti-inflammation effects by which the artificial physiological salt solution has.

In addition, in the case where the artificial physiological salt solution according to the present invention is applied as an artificial infusion, an artificial amniotic fluid or a protective solution, effects can be gained, such that cells and tissue, organs and fetuses are not subjected to oxidation damage, and as a result, recovery becomes faster.

Furthermore, in the case where the artificial physiological salt solution according to the present invention is applied as a cell/tissue culture, cells and tissue are not subjected to oxidation damage with ease, and it becomes possible to implement experiments and the like using cultured cells and cultured tissue in an environment which is close to that within a human body. In addition, particularly in the case where the artificial physiological salt solution according to the present invention is applied as a cell/tissue culture to primary culture cells (cultured cells before being applied to a cell line), significant effects are gained, such that the lifetime of primary culture cells is extended, due to suppression of oxygen damage.

### Brief Description of the Drawings

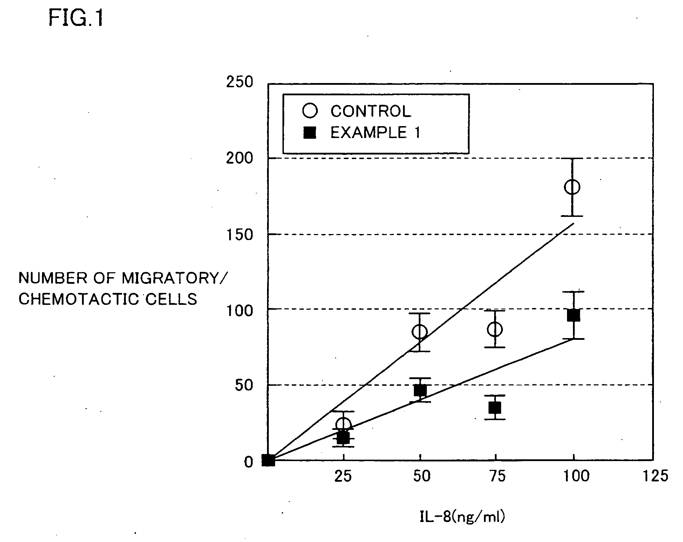

Fig. 1 is a graph showing the activity for suppressing migratory/chemotactic activity in neutrophils due to the artificial physiological salt solution according to the present invention.

### Best Modes for Carrying Out the Invention

An artificial physiological salt solution according to the present invention is characterized by being provided with the active hydrogen reaction value, the pH and the osmotic pressure in respective particular ranges. The artificial physiological salt solution according to the present invention provides anti-inflammation effects by suppressing active oxygen eliminating activity as described below, chemotactic activity and migratory activity of polymorphonuclear leucocytes, in particular, neutrophils, which are inflammatory cells, without negatively affecting organs, tissue and cells to which the artificial physiological salt solution is applied. Here, "artificial physiological salt solution" means a physiological salt solution that has been artificially prepared so as to have a composition which is similar to the body fluids within a human body. In addition, "composition which is similar to the body fluids" means a composition which is close to the composition of the body fluids in organs, tissue or cells, so that, the organ, tissue or cells are not damaged when the artificial physiological salt solution is applied to the organ, tissue or cells, depending on the application. A wide range of applications, such as organ cleaning solutions (for example, cleaning solutions for the eyes (including eye drops) and cleaning solutions for the nasal cavity); artificial infusions, artificial amniotic fluid, protective solutions (for example, protective solutions for myocardia), cell/tissue cultures and the like can be concretely expected for the artificial physiological salt solution of the present invention.

The artificial physiological salt solution according to the present invention has an active hydrogen reaction value (hydrogen radical reaction value) within a range from 0.01 to 1. In the case where the active hydrogen reaction value is less than 0.01 or exceeds 1, the active oxygen eliminating activity and anti-inflammation effects are reduced or lost, and therefore, the object of the present invention cannot be achieved. Though the active hydrogen reaction value in the artificial physiological salt solution according to the present invention is not particularly limited, as long as it is within the above described range, it is preferable for it to be 0.1 to 1, and it is more preferable for it to be 0.5 to 1.

Here, the active hydrogen reaction value in the artificial physiological salt solution according to the present invention is the value that is measured in compliance with the method (hereinafter referred to as "DBNBS method") that was proposed by the present applicant in Japanese Patent Laying-Open No. 2002-350420. The measurement of the active hydrogen reaction value is, concretely, carried out in the following manner.
(1) A hydrogen gas is blown at a constant rate into a solution of 1, 1-diphenyl-2-picryl hydrazyl (DPPH) having absorption in the vicinity of 517 nm in the presence of platinum black, and a graph showing the correlation between attenuation in the degree of absorption in the vicinity of 517 nm and the length of time of blowing in of hydrogen gas is made (formation of calibration curve A).
(2) Not more than 100 µM of cysteine and DPPH are made to react, and a graph showing the correlation between attenuation in absorption in the vicinity of 517 nm in DPPH and the concentration of cysteine is made (formation of calibration curve B).
(3) A hydrogen gas is blown at a constant rate into a solution of sodium 3, 5-dibromo-4-nitrosobenzene sulfonate (DBNBS) having absorption in the vicinity of 450 nm in the presence of platinum black, and a graph showing the correlation between the absorption in the vicinity of 450 nm and the concentration of active hydrogen is made (formation of calibration curve C).
(4) DBNBS is added to a sample (artificial physiological salt solution), which is then heated at 60°C for one hour, and after that, the absorption in the vicinity of 450 nm is measured, and the gained measured value is used as the active hydrogen reacton value (hydrogen radical reaction value).

The pH of the artificial physiological salt solution according to the present invention is within a range from 6.0 to 7.9. In the case where the pH is less than 4.0 or exceeds 7.9, there is a risk that undesirable effects or deterioration in the functions may occur in the organ, tissue or cells to which the artificial physiological salt solution is applied, and the object of the present invention cannot be achieved. Though the pH of the artificial physiological salt solution according to the present invention is not particularly limited, as long as it is within the above described range, it is preferable for it to be 6.0 to 7.7 in the case where, for example, the artificial physiological salt solution according to the present invention is applied as an organ cleaning solution (particularly, it is preferable for the pH to be in a range from 7.3 to 7.6 in the case where the artificial physiological salt solution is used for cleaning the eyes, and it is preferable for the pH to be in a range from 6.0 to 7.4 in the case where it is used for cleaning the nasal cavity). In addition, it is preferable for the pH of the artificial physiological salt solution according to the present invention to be 4.0 to 7.6, it is more preferable for it to be 7.1 to 7.6, and it is most preferable for it to be 7.3 to 7.5 in the case where it is applied as an artificial infusion, an artificial amniotic fluid or a protective solution. In addition, it is preferable for the pH of the artificial physiological salt solution according to the present invention to be 6.9 to 7.5, and it is more preferable for it to be 7.1 to 7.3 in the case where it is applied as a cell/tissue culture.

Here, the pH of the artificial physiological salt solution according to the present invention is the value that is measured by reading the indicator of a pH meter Φ260, made by Beckman, Inc.) when the pH electrodes thereof are immersed in the artificial physiological salt solution.

The osmotic pressure of the artificial physiological salt solution according to the present invention is within a range from 260 mOsm/L to 320 mOsm/L. In the case where the osmotic pressure is less than 260 mOsm/L or exceeds 2560 mOsm/L, there is a risk that undesirable effects or deterioration in the functions may occur in the organ, tissue or cells to which the artificial physiological salt solution is applied, and the object of the present invention cannot be achieved. Though the osmotic pressure of the artificial physiological salt solution according to the present invention is not particularly limited, as long as it is within the above described range, it is preferable for the artificial physiological salt solution according to the present invention to be 270 mOsm/L to 315 mOsm/L, and it is more preferable for it to be 280 mOsm/L to 305 mOsm/L, for example, in the case where it is applied as an organ cleaning solution. In addition, it is preferable for the osmotic pressure of the artificial physiological salt solution according to the present invention to be 270 mOsm/L to 2560 mOsm/L, it is more preferable for it to be 270 mOsm/L to 310 mOsm/L, and it is most preferable for it to be 280 mOsm/L to 300 mOsm/L in the case where it is applied as an artificial infusion, artificial amniotic fluid or a protective solution. In addition, it is preferable for the osmotic pressure of the artificial physiological salt solution according to the present invention to be 260 mOsm/L to 310 mOsm/L, and it is more preferable for it to be 265 mOsm/L to 280 mOsm/L in the case where it is applied as a cell/tissue culture.

Here, the osmotic pressure of the artificial physiological salt solution according to the present invention is the value that is measured on the basis of the principle of freezing point depression method using an osmometer (made by ROEBLING Corporation).

The artificial physiological salt solution according to the present invention, where the active hydrogen reaction value, the pH and the osmotic pressure are respectively within the particular ranges described above provides active oxygen eliminating activity and effects of suppressing chemotactic activity and migratory activity in polymorphonuclear leucocytes, in particular, neutrophils, which are inflammatory cells, and furthermore, does not negatively affect the organ, tissue or cells to which it is applied, and therefore, are appropriate for a multitude of applications as an artificially prepared physiological salt solution.

Here, the active oxygen eliminating activity of the artificial physiological salt solution according to the present invention can be confirmed by means of, for example, a Fenton reaction method. A Fenton reaction method is a method for measuring ·OH from among active oxygen, and is a method where •OH is generated from hydrogen peroxide using iron as a catalyst, luminol, which is a luminous reagent, is excited by •OH, and reduction of active oxygen is observed using light that is emitted at that time. In accordance with a concrete operation method, a highly sensitive chemical luminescence measuring apparatus (CLD-110, made by Tohoku Electronic Industrial Co., Ltd.) is used. A tris-hydrogen chloride buffer solution (pH: 7.8), luminol, DTPA (diethylenetriamine-N, N, N', N", N"'-pentaacetic acid), FeSO₄ and hydrogen peroxide are added to cells so that the final concentrations thereof become 0.1 M, 2.5 µM, 5 µM, 5 µM and 50 µM, respectively, and after that, a sample (artificial physiological salt solution) is added, so that the total amount is adjusted to 2 mL, and then, a change in the intensity of luminescence can be measured and confirmed.

In addition, it is possible to measure the active oxygen eliminating activity of the artificial physiological salt solution according to the present invention in accordance with a hydrogen peroxide method or an HX-XOD method, in addition to the above described Fenton reaction method. The hydrogen peroxide method is a method for measuring H₂O₂ from among active oxygen, and is a method where a reaction solution that is gained by removing a FeSO₄ solution into which DTPA has been mixed from the reaction solution in the above described Fenton reaction method is used, and luminol luminescence originating from hydrogen peroxide is measured. In addition, the HX-XOD method is a method for measuring O₂• from among active oxygen, and is a method where HX (hypoxanthine) is used as stromata, O₂• is generated by means of XOD (xanthine oxidase), O₂• excites CLA (made by Wako Pure Chemical Industries, Ltd.) which is a luminous reagent and reduction in active oxygen that is generated at this time, is observed. Concretely, according to the HX-XOD method, a phosphate buffer solution (pH: 7.8), CLA, XOD and HX are added to cells of CLD-110, so that the final concentrations thereof become 0.2 µM, 1 µM, 5 U/mL and 10 µM, respectively, and after that, a sample (artificial physiological salt solution) is added, so that the total amount is adjusted to 2 mL, and then, the change in the intensity of luminescence is measured, and thereby, active oxygen eliminating activity can be confirmed.

In addition, the effect of attracting inflammatory cells (chemotactic activity and migratory activity) in the artificial physiological salt solution according to the present invention can be confirmed in accordance with a chemotaxis chamber method. Concretely, first, neutrophils that have been separated from human blood are made to float in the artificial physiological salt solution according to the present invention so as to be processed for a constant period of time. After that, a solution of chemokine, such as IL-8 or RANTES, is put in a well in a chamber, and a membrane (polyvinyl pyrrolidone free) made of polycarbonate (pores are slightly smaller than the neutrophils are regularly provided) is mounted on top of this, the above described solution in which the neutrophils float is put on top of this so that it migrates and undergoes chemotaxis for a constant period of time, the neutrophils that have migrated and undergone chemotaxis into the well by passing through the pores of the membrane are dyed with a dying solution, such as Diff-Quick, and counted, and thereby, the effects can be evaluated.

It is preferable for the artificial physiological salt solution according to the present invention to include at least any of sodium ions (Na⁺), potassium ions (K⁺) and chloride ions (Cl⁻). This is because in the case where the artificial physiological salt solution according to the present invention doesn't contain at least any of sodium ions (Na⁺), potassium ions (K⁺) and chloride ions (Cl⁻), cell growth stops in the organ, tissue or cells to which the artificial physiological salt solution is applied, and there is a risk that the functions of the organ, tissue or cells may deteriorate or stop. The artificial physiological salt solution according to a preferable embodiment of the present invention includes at least any of sodium ions, potassium ions and chloride ions, and therefore, effects can be gained, such that the functions of the organ, tissue or cells to which the artificial physiological salt solution is applied can be maintained.

In the case where the artificial physiological salt solution according to the present invention includes sodium ions, it is preferable for the content to be not higher than 200 mEq/L. This is because in the case where the content of sodium ions exceeds 200 mEq/L, there is a risk that undesirable effects or deterioration in the functions may occur in the organ, tissue or cells to which the artificial physiological salt solution is applied. In addition, it is preferable for the artificial physiological salt solution according to the present invention to include not less than 30 mEq/L of sodium ions, from the point of view of preventing deterioration in the functions of the organ, tissue or cells to which the artificial physiological salt solution is applied.

It is preferable for the content of sodium ions in the artificial physiological salt solution according to the present invention to be not higher than 200 mEq/L, as described above in the case where the artificial physiological salt solution according to the present invention is applied as an organ cleaning solution, for example, it is preferable for the content to be 125 mEq/L to 165 mEq/L, and it is more preferable for it to be 135 mEq/L to 155 mEq/L. In addition, in the case where the artificial physiological salt solution according to the present invention is applied as an artificial infusion, artificial amniotic fluid or a protective solution, it is preferable for the content to be 135 mEq/L to 170 mEq/L, and it is more preferable for it to be 145 mEq/L to 160 mEq/L. In addition, in the case where the artificial physiological salt solution according to the present invention is applied as a cell/tissue culture, it is preferable for the content to be 115 mEq/L to 155 mEq/L, and it is more preferable for it to be 125 mEq/L to 145 mEq/L.

In the case where the artificial physiological salt solution according to the present invention includes potassium ions, it is preferable for the content to be not higher than 100 mEq/L. This is because in the case where the content of potassium ions exceeds 100 mEq/L, there is a risk that undesirable effects or deterioration in the functions may occur in the organ, tissue or cells to which the artificial physiological salt solution is applied. In addition, it is preferable for the artificial physiological salt solution according to the present invention to include not less than 1 mEq/L of potassium ions, from the point of view of preventing deterioration in the functions of the organ, tissue or cells to which the artificial physiological salt solution is applied.

It is preferable for the content of potassium ions in the artificial physiological salt solution according to the present invention to be not higher than 100 mEq/L, as described above in the case where the artificial physiological salt solution according to the present invention is applied as an organ cleaning solution, for example, it is preferable for the content to be 10 mEq/L to 40 mEq/L, and it is more preferable for it to be 20 mEq/L to 30 mEq/L. In addition, in the case where the artificial physiological salt solution according to the present invention is applied as an artificial infusion, artificial amniotic fluid or a protective solution, it is preferable for the content to be 2 mEq/L to 10 mEq/L, and it is more preferable for it to be 3 mEq/L to 7 mEq/L. In addition, in the case where the artificial physiological salt solution according to the present invention is applied as a cell/tissue culture, it is preferable for the content to be 1 mEq/L to 8 mEq/L, and it is more preferable for it to be 2 mEq/L to 6 mEq/L.

In the case where the artificial physiological salt solution according to the present invention includes chloride ions, it is preferable for the content to be not higher than 200 mEq/L. This is because in the case where the content of chloride ions exceeds 200 mEq/L, there is a risk that undesirable effects or deterioration in the functions may occur in the organ, tissue or cells to which the artificial physiological salt solution is applied. In addition, it is preferable for the artificial physiological salt solution according to the present invention to include not less than 1 mEq/L of potassium ions, from the point of view of preventing deterioration in the functions of the organ, tissue or cells to which the artificial physiological salt solution is applied.

It is preferable for the content of chloride ions in the artificial physiological salt solution according to the present invention to be not higher than 200 mEq/L, as described above in the case where the artificial physiological salt solution according to the present invention is applied as an organ cleaning solution, for example, it is preferable for the content to be 110 mEq/L to 150 mEq/L, and it is more preferable for it to be 120 mEq/L to 140 mEq/L. In addition, in the case where the artificial physiological salt solution according to the present invention is applied as an artificial infusion, artificial amniotic fluid or a protective solution, it is preferable for the content to be 90 mEq/L to 130 mEq/L, and it is more preferable for it to be 100 mEq/L to 120 mEq/L. In addition, in the case where the artificial physiological salt solution according to the present invention is applied as a cell/tissue culture, it is preferable for the content to be 80 mEq/L to 120 mEq/L, and it is more preferable for it to be 90 mEq/L to 110 mEq/L.

Here, the above described content of sodium ions, potassium ions and chloride ions in the artificial physiological salt solution according to the present invention is the value that is measured using ICP-MS (induced coupling plasma-mass spectrometry) (Agilent 7500, made by Agilent Technologies Corporation).

In addition, the artificial physiological salt solution according to the present invention may contain components other than the above described sodium ions, potassium ions and chloride ions, within a range where the effects of the present invention are not lost. As for these components, amino acids, glucose, citric acid, calcium ions, hydrogen carbonate ions, hydrogen phosphate ions, dihydrogen phosphate ions, glucose, fructose, xylitol, acetic acid, lactic acid and magnesium ions can be cited as examples. In addition to these, in the case where, for example, the artificial physiological salt solution according to the present invention is applied as an organ cleaning solution, sodium chondroitin sulfate, hyaluronic acid and the like can be cited, in the case where the artificial physiological salt solution according to the present invention is applied as an artificial infusion, artificial amniotic fluid or a protective solution, zinc, sulfuric acid, protein, sugars, lipids, phosphorous, iron, iodine, manganese, copper, vanadium, urinary trypsin inhibitors (UTI) and the like can be cited, and in the case where the artificial physiological salt solution according to the present invention is applied as a cell/tissue culture, zinc, sulfuric acid, protein, sugars, lipids, phosphorous, iron, iodine, manganese, copper, vanadium, selenite (sodium selenite), ethanol amine, urinary trypsin inhibitors (UTI), serum, blood plasma, activin and the like can be cited.

It is preferable for adjustment of ion balance to be carried out on electrolytic reduction water in the artificial physiological salt solution according to the present invention. Here, "electrolytic reduction water" is generated in a negative pole chamber through electrolysis, where water (material water) in which an electrolyte is dissolved has been introduced in the negative pole chamber that is separated from a positive pole chamber by a diaphragm, and is water that includes an abundant amount of active hydrogen, which is hydrogen in atomic form having high reactivity. "Electrolytic reduction water" has anti-oxidizing effects because it includes an abundant amount of active hydrogen having reducing (anti-oxidation) properties, and as a result, is useful for preventing or remedying a multitude of diseases, suppressing aging, and in addition, is expected to be used in a multitude of fields, such as food preservation and cleaning of semiconductors, in addition to the medical field. As described above, the above described artificial physiological salt solution according to the present invention can be prepared using electrolytic reduction water, and thereby, an advantage is gained, such that there are no side effects, because the active component of the active substance in the electrolytic reduction water simply returns to water due to its nature, even when active oxygen is eliminated. As for the manufacturing method for electrolytic reduction water, for example, a method using an electrolytic reduction water manufacturing apparatus (TI-8000, made by Nihon Trim Co., Ltd.) is known, and one appropriate method thereof is described below, in the description of a manufacturing method for an artificial physiological salt solution according to the present invention.

In addition, "adjustment of ion balance" means to adjust the content of sodium ions and potassium ions so that potassium ions : sodium ions = 1 : 4 to 1 : 8 (mol ratio). This is because in the case where in the ion balance, potassium ions : sodium ions is less than 1 : 4 or potassium ions : sodium ions exceeds 1 : 8, the functions of the organ, tissue or cells to which the artificial physiological salt solution is applied tend to deteriorate. It is more preferable for the ion balance to be adjusted so that potassium ions : sodium ions = 1 : 5 to 1 : 7 from within the above described range, and it is most preferable for it to be adjusted so that potassium ions : sodium ions = 1 : 6.

Here, whether or not ions are balanced in an artificial physiological salt solution can be confirmed by calculating the mol ratio of the sodium ions of the potassium ions which are contained in the artificial physiological salt solution using ICP-MS (Agilent 7500, made by Agilent Technologies Corporation).

. In addition, it is preferable for the oxidation-reduction potential (ORP) of the artificial physiological salt solution according to the present invention to be within a range from -800 mV to + 200 mV, it is more preferable for it to be within a range from -500 mV to +100mV, and it is most preferable for it to be within a range from -400 mV to +50mV. This is because in the case where the oxidation-reduction potential is less than -800mV or exceeds +200 mV, the functions of the organ, tissue or cells to which the artificial physiological salt solution is applied tend to deteriorate.

Here, the oxidation-reduction potential in the artificial physiological salt solution according to the present invention is a value that is measured using an oxidation-reduction potentiometer (RM-20P, made by Toa Denpa Kogyo Co., Ltd.).

Though the artificial physiological salt solution according to the present invention can be applied to a wide range of applications, it is preferable for it to be applied especially as (a) organ cleaning solutions, (b) artificial infusions, artificial amniotic fluid or protective solutions, and (c) cell/tissue cultures. Here, these merely illustrate applications to which the artificial physiological salt solution according to the present invention is particularly applicable, and the artificial physiological salt solution according to the present invention is widely applicable, without being limited to these applications.

### (a) Application as Organ Cleaning Solution

As for the application as an organ cleaning solution, for example, a cleaning solution for the eyes (including eye drops), a cleaning solution for the nasal cavity, and in addition, applications for cleaning organs, such as skin, the ears, the heart, the lungs, the kidneys, the liver, muscles, the pancreas, the brain, the oral cavity, the abdominal cavity, the spleen, the gallbladder, the appendix and the digestive organs, can be cited.

### (a1) Application as Cleaning Solution for the Eyes (including eye drops)

In inflammation of the eyes, active oxygen is generated in locally inflamed portions, and furthermore, inflamed cells are attracted to these portions, and active oxygen is generated from these attracted cells in such a manner that these two phenomena cause itchiness in the eyes and symptoms such as asthenopia, and damage eye tissue. In order to prevent damage by active oxygen as described above, the ability to eliminate active oxygen and the ability to make it difficult for inflammatory cells to be attracted to inflamed portions is necessary. The artificial physiological salt solution according to the present invention exhibits active oxygen eliminating activity and effects of suppressing chemotactic activity and migratory activity in polymorphonuclear leucocytes, in particular, neutrophils, which are inflammatory cells, and therefore, in the case where the artificial physiological salt solution according to the present invention is used as a cleaning solution for the eyes and for cleaning eyes, preventing eye disease (after swimming, when dust or sweat get into the eyes, when contact lenses are used, during work on VDT and the like), and used as a cleaning solution during healing of the eyes and at the time of operation, inflammation is suppressed, due to the anti-inflammation effects of the electrolytic reduction water, making it difficult for the tissue in the eyes to be damaged by active oxygen or substances that generates active oxygen. As a result of this, symptoms such as dry eyes, strange or uncomfortable feelings due to a foreign body, tired eyes, itchiness of the eye, an increase in blinking, and bloodshot eyes can be alleviated. Accordingly, the artificial physiological salt solution according to the present invention is particularly appropriate for use as a cleaning solution for the eyes (including eye drops) which is used for cleaning eyes in order to prevent eye tissue from being damaged by active oxygen that is generated at the time of dry eyes, use of contact lenses, dry eyes resulting from VDT work, strange or uncomfortable feelings due to a foreign body, tired eyes, itchiness of the eye, an increase in blinking, and bloodshot eyes, for preventing eye disease (after swimming, when dust or sweat gets into the eyes), or at the time of healing of the eyes and at the time of operation. In recent clinical research on dry eyes, 31.2 % of VDT workers had dry eyes, and 44 % were suspected to have dry eyes. In recent years, the number of VDT workers has been steadily increasing, and actually, a large number of people are suffering from dry eyes or symptoms that are close to this. It is possible to manufacture the artificial physiological salt solution according to the present invention in lines, and it is possible to mass produce with a high level of reproducibility, and therefore, it is possible to widely provide the artificial physiological salt solution according to the present invention to the people who are suffering from dry eyes or symptoms that are close to this.

In the case where the artificial physiological salt solution according to the present invention is used as a cleaning solution for the eyes, the artificial physiological salt solution is contained, for example, in a cup of 5 mL to 20 mL with the rim in a form that fits around an eye, and the rim is pressed against the area around the eye, and the user blinks with the artificial physiological salt solution making contact with the eye, and thereby, the eye can be cleaned. In-addition, the artificial physiological salt solution according to the present invention can be used in the form of eye drops, and in this case, the artificial physiological salt solution is contained within a conventional container for eye drops that is used to allow several eye drops to fall into the eyes. In the case where it is used in eye drops, it is preferable for the eye drops to be in disposable type packs, without mixing in an antiseptic as in conventional eye drops.

### (a-2) Application as Cleaning Solution for Nasal Cavity

In addition, in the case where the artificial physiological salt solution according to the present invention is applied as a cleaning solution for the nasal cavity, dust, sundry germs, pollen and the like within the nasal cavity can be removed, and the inside of the nasal cavity can be cleaned appropriately, and in addition, allergy symptoms such as hay fever can be suppressed, due to the anti-oxidation effects and anti-inflammation effects of the artificial physiological salt solution, without causing undesired side-effects. In the case where the artificial physiological salt solution according to the present invention is used as a cleaning solution for the nasal cavity, for example, it is contained within a container having a spraying means so as to provide a spray form that is appropriate for spraying into the nasal cavity, and thereby, the nasal cavity can be cleaned appropriately.

### (b) Application as Artificial Infusion, Artificial Amniotic Fluid or Protective Solution

In the case where the artificial physiological salt solution according to the present invention is used as an artificial infusion, artificial amniotic fluid or a protective solution (for example, protective solution for myocardia), the cells or the tissue, the organ or the fetus is not subjected to oxidation damage with ease, and as a result, effects can be gained, where recovery becomes faster. In the case where the artificial physiological salt solution according of the present invention is used as an artificial infusion, the artificial physiological salt solution can be provided in any well-known form, for example, contained within an infusion bag. In addition, in order to prevent edema by reducing the amount for administration of an infusion, an artificial infusion may be implemented as a concentrated solution (having a concentration that is, for example, 8 times higher than conventional artificial physiological salt solutions) having a high osmotic pressure within the above described range in the present invention. In the case where the artificial physiological salt solution according to the present invention is used as an artificial amniotic fluid, it can be provided in such a form as to be contained in an appropriate container in such a manner that it can be injected into the uterus of a pregnant woman who is suffering from amniorrhexis at the time of premature delivery, or oligohydramnios transvaginally using, for example, a catheter, at the site of giving birth. In addition, in the case where the artificial physiological salt solution according to the present invention is used as a protective solution (for example, protective solution for myocardia), the artificial physiological salt solution can, for example, be provided in such a form as to be contained within any well-known infusion bag.

### (c) Application as Cell/Tissue Culture

In the case where the artificial physiological salt solution according to the present invention is used as a cell/tissue culture, effects can be gained, such that cells or tissue is not subjected to oxidation damage with ease. As a result of this, it becomes possible to conduct experiments using cells and tissue which is cultured in an environment that is close to that within the human body. As for the cells that can be cultured in the artificial physiological salt solution according to the present invention, a multitude of well-known culture cells, such as primary culture cells (cultured cells before being applied to a cell line), passage culture cells and ES cells, can be cited. From among these, in the case where the artificial physiological salt solution according to the present invention is used for culturing primary culture cells, notable effects can be gained, such that the lifetime of the primary culture cells is extended, due the suppression of oxidation damage. In the case where the artificial physiological salt solution according to the present invention is used as a cell/tissue culture, it can be provided in such a state as to be condensed in advance and contained within a container, and then, mixed for use, on the basis of the application, with an appropriate culture medium solution with a desired ratio that is appropriate for culturing cells/tissue.

The artificial physiological salt solution according to the present invention may contain a multitude of proteins, within a range where the effect of the present invention are not lost. As these proteins, insulin, transferrin, superoxide dismutase (SOD), thioredoxin, glutathione peroxidase, a urinary trypsin inhibitor (UTI), albumin and activin can be cited as examples. It is preferable for the content of the above described protein to be not higher than 6.0 mass % in the artificial physiological salt solution according to the present invention. This is because in the case where the content of the protein exceeds 6.0 mass %, there is a risk that undesirable effects or deterioration in the function may occur in the organ or cells to which the artificial physiological salt solution is applied.

The present invention also provides a method for manufacturing an artificial physiological salt solution according to the present invention, as described above. A manufacturing method for an artificial physiological salt solution according to the present invention is a method for adjusting electrolytic reduction water so that the active hydrogen reaction value becomes 0.01 to 1, the pH becomes 6.0 to 7.9, and the osmotic pressure becomes 260 mOsm/L to 320 mOsm/L.

In accordance with the manufacturing method according to the present invention, first, electrolytic reduction water is gained using, for example, an electrolytic reduction water manufacturing apparatus (TI-8000, made by Nihon Trim Co., Ltd.). Electrolytic reduction water can be gained by, for example, adding an appropriate electrolyte to a material water (water before being electrolyzed), and after that, electrolyzing the water. As for the material water that is used for the preparation of electrolytic reduction water, ultra-pure water (MilliQ water), pure water, distilled water and faucet water can be cited as examples, and it is preferable to use ultra-pure water, pure water or distilled water as the material water, taking into consideration the fact that it is to be applied to a human body or living cells.

As for the electrolyte, sodium hydroxide, potassium hydroxide, sodium chloride, potassium chloride and hexachloroplatinic acid can be cited as examples, and from among these, sodium hydroxide and/or potassium hydroxide is preferable, in order to prevent mixture of hypochlorous acid that is generated from the positive pole, as well as the occurrence of hypochlorous acid. The electrolyte is added to the material water in such a manner that the electric conductivity of the material water becomes, preferably, not less than 100 µS/cm, and more preferably, 100 µS/cm to 1000 µS/cm. The added amount of electrolyte needs, in order to gain such an electric conductivity in the material water, to be, for example, 4 mg/L to 800 mg/L, preferably, 10 mg/L to 300 mg/L, and more preferably 50 mg/L to 200 mg/L, in the case where sodium hydroxide is used as the electrolyte

Electrolysis can be carried out using a well-known electrolytic reduction water manufacturing apparatus (preferably TI-8000 (made by Nihon Trim Co., Ltd.)), that is provided with at least an electrolytic bath having a negative pole chamber that includes a negative pole and a positive pole chamber that includes a positive pole which are separated from each other by a diaphragm. The conditions for electrolysis are not particularly limited, and electrolysis can be carried out under conventionally known appropriate conditions. Conditions where, for example, the current is 1 A to 5 A, the voltage is 0 V to 80 V, the temperature is 4°C to 60°C, the length of time is 1 second to one hour, and the flow rate is 0 mL/min to 1500 mL/min, can be cited. After the completion of electrolysis, the water that has been produced in the negative pole chamber can be provided as electrolytic reduction water. Furthermore, it is preferable to provide the electrolytic reduction water after carrying RO (reverse osmosis membrane) processing.

In accordance with the manufacturing method for an artificial physiological salt solution according to the present invention, electrolytic reduction water that has been gained in this manner is adjusted so that the active hydrogen reaction value, the pH and the osmotic pressure respectively fall within the above described range. The active hydrogen reaction value, for example, can be adjusted by diluting or condensing the electrolytic reduction water so that a desired active hydrogen reaction value is gained. In addition, the pH can be adjusted by adding a buffer agent (for example, sodium hydrogen carbonate, dibasic sodium phosphate, sodium dihydrogen phosphate, citric acid, sodium citrate and HEPES), or by adding hydrochloric acid or sodium hydroxide. In addition, the osmotic pressure can be adjusted, for example, by diluting or condensing the electrolytic reduction water so that a desired osmotic pressure is gained. These techniques may be appropriately combined for adjustment taking the mutual effects into consideration, so that the active hydrogen reaction value finally becomes 0.01 to 1, the pH becomes 6.0 to 7.9, and the osmotic pressure becomes 260 mOsm/L to 320 mOsm/L.

In accordance with the manufacturing method for an artificial physiological salt solution according to the present invention, it is preferable for the step of adjusting the ion balance in the electrolytic reduction water by adding sodium chloride and/or potassium chloride to further be included. Concretely, the content of sodium ions and potassium ions can be adjusted by adding sodium chloride and/or potassium chloride, so that potassium ions : sodium ions = 1 : 4 to 1 : 8 (mol ration) is gained in the electrolytic reduction water. Here, sodium chloride and potassium chloride are used, because they do not affect the pH.

This step of adjusting the ion balance may be carried out before or after the adjustment of the above described active hydrogen reaction value, the pH and the osmotic pressure, but it is preferable for it to be carried out before the adjustment of the active hydrogen reaction value, the pH and the osmotic pressure, because these ions are used also at the time of adjustment of the osmotic pressure.

It is preferable for all of the steps in the manufacturing method for an artificial physiological salt solution according to the present invention to be carried out in an aseptic state, or for sterilization of the filter or sterilization using an autoclave to be carried out after the preparation of the artificial physiological salt solution.

Here, the artificial physiological salt solution according to the present invention is not limited to that which is manufactured in accordance with the above described manufacturing method according to the present invention, as long as the active hydrogen reaction value is 0.01 to 1, the pH is 6.0 to 7.9, and the osmotic pressure is 260 mOsm/L to 320 mOsm/L, as described above. The artificial physiological salt solution according to the present invention can also be manufactured, for example, in accordance with a method where a hydrogen absorbing/absorbing metal colloid selected from the group consisting of a platinum colloid, a palladium colloid, a vanadium colloid, an iron colloid and a silica colloid is added to water, and after that, dissolved hydrogen is generated by means of hydrogen gas bubbling, dissolving of minerals, ultrasonic processing, magnetizing processing, physical inheritance, atomic vibration using microwaves, light radiation or the like, and then, the active hydrogen reaction value, the pH and the osmotic pressure are appropriately adjusted, or in accordance with a method where lithium, sodium or magnesium is dissolved in acid water, and after that, the active hydrogen reaction value, the pH and the osmotic pressure are appropriately adjusted. It is preferable, however, for the artificial physiological salt solution according to the present invention to be manufactured in accordance with the above described manufacturing method according to the present invention.

In the following, the present invention is described in further detail by citing examples, but the present invention is not limited to these.

### <Example 1>

A mixed solution of 0.857 mM of an NaOH aqueous solution and 0.143 mM of a KOH aqueous solution (mixed solution of 0.428 mL of 2 N NaOH aqueous solution and KOH aqueous solution (0.428mL of 2 N NaOH aqueous solution and 0.072 mL of 2 N KOH are dissolved in one liter of MiliQ water)) was electrolyzed in an electrolytic reduction water manufacturing apparatus (TI-8000, made by Nihon Trim Co., Ltd.) (current: 5 A, temperature: 20°C, flow rate: 1100 mL/min). A 1.437M NaCl-238 mM KCl aqueous solution was added to the produced electrolytic reduction water in order to adjust the osmotic pressure (mixed ratio was NaCl-KCl aqueous solution: electrolytic reduction water = 1:9). Then, the pH was adjusted to 7.5 using 1 N HCl and 0.1 N HCl.

As a result, an artificial physiological salt solution of which the active hydrogen reaction value was 0.06 (measured in accordance with a DBNBS method), the pH was 7.5 (measured using a pH meter (Φ 260, made by Beckman Inc.)) and the osmotic pressure was 303 mOsm/L (measured using an osmometer (made by ROEBLING Corporation)) was gained. This artificial physiological salt solution contained 145 mEq/L of sodium ions, 24 mEq/L of potassium ions and 170 mEq/L of chloride ions (respectively measured using ICP-MS (Agilent 7500, made by Agilent Technologies Corporation)), and the oxidation-reduction potential was -100 mV (measured using an oxidation-reduction potentiometer (RM-20P, made by Toa Denpa Kogyo Co., Ltd.)).

### <Comparative Example 1>

An artificial physiological salt solution was prepared in the same manner as in Example 1, except that distilled water was used instead of electrolytic reduction water. The gained artificial physiological salt solution was measured in the same manner as in Example 1, and it was found that the active hydrogen reaction value was 0, the pH was 7.5, and the osmotic pressure was 303 mOsm/L. In addition, the content of sodium ions, which was measured in the same manner as Example 1, was 145 mEq/L, the content of potassium ions was 24 mEq/L, the content of chloride ions was 170 mEq/L, and the oxidation-reduction potential was +350 mV.

### <Comparative Example 2>

An artificial physiological salt solution was prepared in the same manner as in Example 1, except that the electrolytic reduction water that was prepared in Example 1 was condensed to five times its original concentration for use instead of the original electrolytic reduction water. The gained artificial physiological salt solution was measured in the same manner as in Example 1, and it was found that the active hydrogen reaction value was 1.8, the pH was 7.6, and the osmotic pressure was 303 mOsm/L. In addition, the content of sodium ions, which was measured in the same manner as Example 1, was 145 mEq/L, the content of potassium ions was 24 mEq/L, the content of chloride ions was 180 mEq/L, and the oxidation-reduction potential was -150 mV.

### <Comparative Example 3>

An artificial physiological salt solution was prepared in the same manner as in Example 1, except that the pH of the electrolytic reduction water that was prepared in Example 1 was adjusted to 5.5 by adding 1 N HCl. The gained artificial physiological salt solution was measured in the same manner as in Example 1, and it was found that the active hydrogen reaction value was 0.06, the pH was 5.5, and the osmotic pressure was 303 mOsm/L. In addition, the content of sodium ions, which was measured in the same manner as Example 1, was 145 mEq/L, the content of potassium ions was 24 mEq/L, the content of chloride ions was 200 mEq/L, and the oxidation-reduction potential was -50 mV.

### < Comparative Example 4>

An artificial physiological salt solution was prepared in the same manner as in Example 1, except that the pH of the electrolytic reduction water that was prepared in Example 1 was adjusted to 8.5 by adding 1 N HCl. The gained artificial physiological salt solution was measured in the same manner as in Example 1, and it was found that the active hydrogen reaction value was 0.06, the pH was 8.5, and the osmotic pressure was 304 mOsm/L. In addition, the content of sodium ions, which was measured in the same manner as Example 1, was 145 mEq/L, the content of potassium ions was 24 mEq/L, the content of chloride ions was 180 mEq/L, and the oxidation-reduction potential was -100 mV.

### <Comparative Example 5>

An artificial physiological salt solution was prepared in the same manner as in Example 1, except that a 1.437 M NaCl-238 mM KCl aqueous solution was mixed into electrolytic reduction water at the time of the adjustment of the osmotic pressure (mixed ratio was NaCl-KCl aqueous solution : electrolytic reduction water = 0.8 : 9.2). The gained artificial physiological salt solution was measured in the same manner as in Example 1, and it was found that the active hydrogen reaction value was 0.06, the pH was 7.5, and the osmotic pressure was 240 mOsm/L. In addition, the content of sodium ions, which was measured in the same manner as Example 1, was 115 mEq/L, the content of potassium ions was 19 mEq/L, the content of chloride ions was 135 mEq/L, and the oxidation-reduction potential was -50 mV.

### <Comparative Example 6>

An artificial physiological salt solution was prepared in the same manner as in Example 1, except that a 1.437 M NaCl-238 mM KCl aqueous solution was mixed into electrolytic reduction water at the time of the adjustment of the osmotic pressure (mixed ratio was NaCl-KCl aqueous solution : electrolytic reduction water = 1.2 : 8.8). The gained artificial physiological salt solution was measured in the same manner as in Example 1, and it was found that the active hydrogen reaction value was 0.06, the pH was 7.5, and the osmotic pressure was 360 mOsm/L. In addition, the content of sodium ions, which was measured in the same manner as Example 1, was 175 mEq/L, the content of potassium ions was 30 mEq/L, the content of chloride ions was 205 mEq/L, and the oxidation-reduction potential was -50 mV.

### <Evaluation Test 1>

### Evaluation Test for Active Oxygen Eliminating Activity

The measured value of active oxygen (cpm) in the artificial physiological salt solution in Example 1 and Comparative Examples 1 to 6 was calculated in accordance with a hydrogen peroxide method, using luminol luminescence originating from hydrogen peroxide. The results are shown in Table 1.

**Table 1**

| | Example 1 | Comparative Example 1 | Comparative Example 2 | Comparative Example 3 | Comparative Example 4 | Comparative Example 5 | Comparative Example 6 |
|---|---|---|---|---|---|---|---|
| Active hydrogen reaction value | 0.06 | 0 | 1.8 | 0.06 | 0.06 | 0.06 | 0.06 |
| pH | 7.5 | 7.5 | 7.6 | 5.5 | 8.5 | 7.5 | 7.5 |
| Osmotic pressure (mOsm/L) | 303 | 303 | 303 | 303 | 304 | 240 | 360 |
| Na⁺(mEq/L) | 145 | 145 | 145 | 145 | 145 | 115 | 175 |
| K⁺(mEq/L) | 24 | 24 | 24 | 24 | 24 | 19 | 20 |
| Cl⁻(mEq/L) | 170 | 170 | 180 | 200 | 180 | 135 | 205 |
| Oxidation-reduction potential (mV) | -100 | +350 | -150 | -50 | -100 | -50 | -50 |
| Evaluation Test 1 | 597979 | 1917063 | 683433 | 614453 | 625025 | 619432 | 634282 |
| (Measured value of active | ±34903 | ±59565 | ±22323 | ±32239 | ±89892 | ±23898 | ±45292 |
| oxygen (cpm)) Evaluation Test 2 (Number of migratory cells (when IL-8 is 100 ng/ml) | 95±21 | 180±23 | 135±15 | - | - | - | - |
| Evaluation Test 2 (survival rate (%)) | 98 | 98 | 97 | 60 | 49 | 47 | 39 |
| Evaluation Test 3 (feel of use of cleaning solution for eyes) | Good | Good /Passable | Good/Passable | Bad | Bad | Bad | Bad |
| Evaluation Test 4 (TBARS (nmol/ml)) | 2.7±0.5 | 4.6±0.5 | 2.3±0.4 | 3.1±0.4 | - | - | - |
| Evaluation Test 5 (survival ratio (%) after culturing for 24 hours) | 78 | 41 | 80 | 10 | 2 | 0 | 0 |

### <Evaluation Test 2>

### Evaluation Test for Effect of Suppressing Migratory Activity/Chemotactic Activity of Neutrophils

Fig. 1 shows the results of an experiment that was conducted for the purpose of clarifying whether the artificial physiological salt solution according to the present invention suppresses the migratory activity/chemotactic activity of human neutrophils due to chemokine. As for the method for experiment, a chemotaxis chamber method according to which the migratory activity/chemotactic activity of inflammatory cells can be measured was used. First, neutrophils that were separated from human blood were made to float in the artificial physiological salt solution of Example 1 and processed for a constant period of time. A solution of chemokine (IL-8, RANTES or the like) was put in a well of a chamber, and a membrane was placed on top of this. Pores which are slightly smaller than the size of the neutrophils were regularly provided in this membrane. After assembling the chamber, the solution in which neutrophils were floating was put in the well on top of this membrane and made to migrate/undergo chemotaxis for a constant period of time. After that, cells which migrated/undergone chemotaxis through the pores of the membrane were dyed with a special dying solution (Diff-QuiK) and counted. The gained number of migratory cells (when IL-8 was 100 ng/ml) is shown in Table 1.

As is clear from Fig. 1 and Table 1, the number of cells that migrated and underwent chemotaxis in the neutrophils that were processed with the artificial physiological salt solution according to Example 1 of the present invention was approximately half of that of the control (cleaning water prepared from distilled water), and thus, it can be seen that the artificial physiological salt solution according to the present invention suppresses the migratory/chemotactic activity of neutrophils. In addition, the same tests as those described above were carried out on Comparative Examples 1 to 6, and as a result, no suppressing activity was observed in Comparative Example 1, as in the control. Though suppressing activity was observed in Comparative Example 2, the amount was approximately half of that in the example. The survival rate of the cells was not higher than half in Comparative Examples 3 to 6, and measurement was impossible.

In addition, at the same time as the above described tests, effects of the artificial physiological salt solution on neutrophils were confirmed in accordance with a living cell measuring method. As for the living cell measuring method, concretely, trypan blue testing was used. Living cells get rid of trypan blue (blue pigment) to the outside, and therefore, the cells are not dyed, while dead cells can not do this and are dyed. Trypan blue testing is a method where the number of dyed cells and the number of non-dyed cells are counted through a phase contrast microscope, and the survival rate (number of non-dyed cells ÷ (number of dyed cells + number of non-dyed cells) × 100) (%) is calculated. The results are shown in Table 1. As shown in Table 1, it was confirmed that the neutrophils were not at all damaged by the artificial physiological salt solution according to Example 1 of the present invention. Meanwhile, in the case of Comparative Examples 3 to 6, the survival rate was not higher than half. Accordingly, it was found that it is not appropriate to use cells in such a state for migratory testing.

### <Evaluation Test 3>

Evaluation of Feel of Use in the Case of Use as Cleaning Solution for Eyes

The artificial physiological salt solution of Example 1 was used, and eye cleaning was carried out on the inventor's eyes, and there was no uncomfortable stimulation, and dust was captured in the solution after cleaning. In addition, ten volunteers carried out eye cleaning in the same manner and made the same evaluation, in accordance with the following three levels.

good: eye cleaning could be carried out without any uncomfortable stimulation in the eyes

passable: eye cleaning could be carried out, but there was some uncomfortable stimulation in the eyes, or though there was no uncomfortable stimulation in the eyes, eye cleaning could not be carried out

bad: there was uncomfortable stimulation in the eyes, and eye cleaning could not be carried out

The results are shown in Table 1. As shown in Table 1, all of the volunteers made the evaluation that eye cleaning could be carried out without any uncomfortable stimulation in the eyes. As a result of this, it can be seen that the artificial physiological salt solution according to the present invention has eye cleaning effects. Meanwhile, for Comparative Examples 1 and 2, half of the evaluations were good and half were passable, and thus, there was inconsistency in the evaluation. For Comparative Examples 3 to 6, all of the evaluations were bad, and there was uncomfortable stimulation in the eyes.

### <Evaluation Test 4>

### Evaluation as Artificial Infusion

The artificial physiological salt solution (Example 1) that was prepared from electrolytic reduction water was continuously administrated as an infusion into a vein of a rat to which oxidation stress was provided using FNA (ferric nitrilotriacetic acid), and the effect of improvement against oxidation stress was evaluated. TBARS (thiobarbituric acid-reactive substances) was used as an indicator of oxidation stress. This is used to measure a red substance that is generated by making malondialdehyde or alkylaldehyde that is generated through the decomposing reaction of a peroxidized lipid that is generated through the peroxidation reaction of an unsaturated aliphatic acid, the decomposing reaction of a sugar, or the decomposing reaction of a nucleic acid base react with thiobarbituric acid.

First, 6 week-old SD based male rats were divided into four groups ((1) processing with FNA for 0 hours, (2) processing with FNA for 6 hours/administration of artificial physiological salt solution of Example 1, (3) processing with FNA for 6 hours/administration of artificial physiological salt solution of Comparative Example 1, (4) processing with FNA for 6 hours/administration of artificial physiological salt solution of Comparative Example 2) in order to carry out the test. A cannula for continuously administrating an artificial physiological salt solution was attached to the external jugular vein of each rat three days before the collection of blood. After the attachment, the cannula was cleaned everyday with a physiological salt solution containing 500 u/ml of heparin in order to prevent clogging.

FNA was administered into the abdominal cavity so that each rat had 10 mg Fe/kg. Immediately thereafter, a syringe pump MODEL 11 (made by Harvard Apparatus Corporation) was used, and the artificial physiological salt solution of Example 1, Comparative Example 1 and Comparative Example 2 was continuously administered to the rats in the respective groups via the cannula for one hour at a flow rate of 0.05 ml/min. 6 hours after the administration of FNA into the abdominal cavity, all of the blood was collected from an artery in the abdomen by opening the abdominal area under anesthesia using ether. The blood plasma was frozen at -80°C and preserved until the measurement of TBARS. The amount of TBARS in the rat blood plasma was measured using a TBARS assay kit OXLtec (made by ZeptoMetrix Corporation) in accordance with the protocol thereof. The results are shown in Table 1.

As a result, 1.5 +/- 0.3 nmol/ml, which is the TBARS value in the case of being processed with FNA for 0 hours, increased to 4.6 ± 0.5 nmol/ml, which is 3.1 times higher, as a result of processing with FNA for 6 hours in Comparative Example 1, while it only increased to 2.7 ± 0.5 nmol/1, which is 1.8 times higher, in Example 1. This means that oxidation stress due to FNA was reduced by 42 %. In Comparative Example 2, the effects did not increase along with the ratio of condensation of electrolytic reduction water, and the TBARS value only increased to 2.3 ± 0.4 nmol, which is 1.5 times higher. This means that oxidation stress due to FNA was reduced by 52 %. Here, cell toxicity was observed in the above described Evaluation Test 2, and therefore, no evaluation test was carried out for Comparative Examples 4 to 6.

### <Evaluation Test 5>

### Effects as Cell/Tissue Culture

Neutrophils separated from human blood were primarily cultured in the artificial physiological salt solution according to the present invention, and whether or not the survival rate increases was evaluated. In the evaluation, neutrophils separated from human blood were suspended in the artificial physiological salt solution of Example 1 and Comparative Examples 1 to 6, planted in 5 ml laboratory dishes for culturing, and primarily cultured for 24 hours in an incubator with 5 % of carbon dioxide gas at 37°C, and the survival ratio was calculated in accordance with a trypan blue test. The results are shown in Table 1.

At the beginning of culturing, the survival rate was 98 %, while, as can be seen from Table 1, the survival rate in Comparative Example 1 after 24 hours of culturing was reduced to 41 %. Meanwhile, the survival rate in Example 1 was a high value, that is, 78 %, though the survival rate was reduced. This indicates that the artificial physiological salt solution of Example 1 contributed to the increase of the survival rate in the culture of primary cells of which the survival rate was low. In addition, though Comparative Example 2 did not contribute to the increase in the survival rate along with the ratio of condensation of electrolytic reduction water, a high survival rate of as high as 80 % was maintained. In addition, as for the other survival ratios, Comparative Example 3 was 10 %, Comparative Example 4 was 2 %, Comparative Example 5 was 0 %, and Comparative Example 6 was 0 %.

## Claims

1. An artificial physiological salt solution which can be used as an organ cleaning solution, wherein the active hydrogen reaction value that is measured in compliance with the DBNBS method using sodium 3, 5-dibromo-4-nitrosobenzene sulfonate (DBNBS) is 0.01 to 1, the pH is 6.0 to 7.9 and the osmotic pressure is 260 mOsm/L to 320 mOsm/L.

2. The artificial physiological salt solution according to Claim 1, **characterised by** including sodium ions, potassium ions and chloride ions.

3. The artificial physiological salt solution according to Claim 2, **characterised by** including not higher than 200mEq/L of sodium ions.

4. The artificial physiological salt solution according to Claim 2, **characterised by** including not higher than 100 mEq/L of potassium ions.

5. The artificial physiological salt solution according to Claim 2, **characterised by** including not higher than 200 mEq/L of chloride ions.

6. The artificial physiological salt solution according to Claim 1, wherein adjustment of ion balance is carried out on electrolytic reduction water.

7. The artifical physiological salt solution according to Claim 1, **characterised in that** the oxidation reduction potential is -800 mV to +200 mV.

8. A manufacturing method for an artificial physiological salt solution according to Claim 1, **characterised by** adjusting electrolytic reduction water by diluting or condensing and/or adding at least one selected from the group consisting of a buffer agent, hydrochloric acid and sodium hydroxide so that the active hydrogen reaction value that is measured in compliance with the DBNBS method using sodium 3, 5-dibromo-4-nitrosobenzene sulfonate (DBNBS) becomes 0.01 to 1, the pH becomes 6.0 to 7.9 and the osmotic pressure becomes 260 mOsm/L to 320 mOsm/L.

9. The manufacturing method for an artificial physiological salt solution according to Claim 8, **characterised by** further comprising the step of adjusting ion balance in electrolytic reduction water by adding sodium chloride and/or potassium chloride.

10. An artificial physiological salt solution wherein the active hydrogen reaction value that is measured in compliance with the DBNBS method using sodium 3, 5-dibromo-4-nitrosobenzene sulfonate (DBNBS) is 0.01 to 1, the pH is 6.0 to 7.9 and the osmotic pressure is 260 mOsm/L to 320 mOsm/L, for use as an organ cleaning solution.

11. Use of an artificial physiological salt solution wherein the active hydrogen reaction value that is measured in compliance with the DBNBS method using sodium 3, 5-dibromo-4-nitrosobenzene sulfonate (DBNBS) is 0.01 to 1, the pH is 6.0 to 7.9 and the osmotic pressure is 260 mOsm/L to 320 mOsm/L, for the manufacture of an organ cleaning solution.

## Patentansprüche

1. Künstliche physiologische Salzlösung, die als Organreinigungslösung verwendet werden kann, wobei der Reaktionswert von aktivem Wasserstoff, gemessen gemäß dem DBNBS-Verfahren unter Verwendung von Natrium-3,5-dibrom-4-nitrosobenzolsulfonat (DBNBS), 0,01 bis 1 beträgt, der pH-Wert 6,0 bis 7,9 beträgt und der osmotische Druck 260 mosm/l bis 320 mOsm/l beträgt.

2. Künstliche physiologische Salzlösung gemäß Anspruch 1, **gekennzeichnet durch** Umfassen von Natriumionen, Kaliumionen und Chloridionen.

3. Künstliche physiologische Salzlösung gemäß Anspruch 2, **gekennzeichnet durch** Umfassen von nicht mehr als 200 mÄq/l Natriumionen.

4. Künstliche physiologische Salzlösung gemäß Anspruch 2, **gekennzeichnet durch** Umfassen von nicht mehr als 100 mÄq/l Kaliumionen.

5. Künstliche physiologische Salzlösung gemäß Anspruch 2, **gekennzeichnet durch** Umfassen von nicht mehr als 200 mÄq/l Chloridionen.

6. Künstliche physiologische Salzlösung gemäß Anspruch 1, wobei das Einstellen des Ionengleichgewichts an elektrolytreduziertem Wasser durchgeführt wird.

7. Künstliche physiologische Salzlösung gemäß Anspruch 1, **dadurch gekennzeichnet, dass** das Oxidationsreduktionspotential -800 mV bis +200 mV beträgt.

8. Herstellungsverfahren für eine künstliche physiologische Salzlösung gemäß Anspruch 1, **gekennzeichnet durch** Einstellen von elektrolytreduziertem Wasser **durch** Verdünnen oder Kondensieren und/oder Zugeben von wenigstens einem, ausgewählt aus der Gruppe, bestehend aus einem Puffermittel, Salzsäure und Natriumhydroxid, so dass der Reaktionswert von aktivem Wasserstoff, gemessen gemäß dem DBNBS-Verfahren unter Verwendung von Natrium-3,5-dibrom-4-nitrosobenzolsulfonat (DBNBS), 0,01 bis 1 wird, der pH-Wert 6,0 bis 7,9 wird und der osmotische Druck 260 mosm/l bis 320 mOsm/l wird.

9. Herstellungsverfahren für eine künstliche physiologische Salzlösung gemäß Anspruch 8, **gekennzeichnet durch** zusätzliches Umfassen des Schritts des Einstellens des Ionengleichgewichts in elektrolytreduziertem Wasser **durch** Zugeben von Natriumchlorid und/oder Kaliumchlorid.

10. Künstliche physiologische Salzlösung, wobei der Reaktionswert von aktivem Wasserstoff, gemessen gemäß dem DBNBS-Verfahren unter Verwendung von Natrium-3,5-dibrom-4-nitrosobenzolsulfonat (DBNBS), 0,01 bis 1 beträgt, der pH-Wert 6,0 bis 7,9 beträgt und der osmotische Druck 260 mOsm/l bis 320 mOsm/l beträgt, zur Verwendung als Organreinigungslösung.

11. Verwendung einer künstlichen physiologischen Salzlösung, wobei der Reaktionswert von aktivem Wasserstoff, gemessen gemäß dem DBNBS-Verfahren unter Verwendung von Natrium-3,5-dibrom-4-nitrosobenzolsulfonat (DBNBS), 0,01 bis 1 beträgt, der pH-Wert 6,0 bis 7,9 beträgt und der osmotische Druck 260 mOsm/l bis 320 mOsm/l beträgt, für die Herstellung einer Organreinigungslösung.

## Revendications

1. Solution saline physiologique artificielle qui peut être utilisée comme solution de nettoyage d'organe, **caractérisée en ce que** la valeur de réaction d'hydrogène actif qui est mesurée conformément à la méthode de DBNBS en utilisant du 3,5-dibromo-4-nitrosobenzènesulfonate de sodium (DBNBS) est de 0,01 à 1, le pH est de 6,0 à 7,9 et la pression osmotique est de 260 mOsm/L à 320 mOsm/L.

2. Solution saline physiologique artificielle selon la revendication 1, **caractérisée en ce qu'**elle comprend des ions sodium, des ions potassium et des ions chlorure.

3. Solution saline physiologique artificielle selon la revendication 2, **caractérisée en ce qu'**elle comprend au plus 200 mEq/L d'ions sodium.

4. Solution saline physiologique artificielle selon la revendication 2, **caractérisée en ce qu'**elle comprend au plus 100 mEq/L d'ions potassium.

5. Solution saline physiologique artificielle selon la revendication 2, **caractérisée en ce qu'**elle comprend au plus 200 mEq/L d'ions chlorure.

6. Solution saline physiologique artificielle selon la revendication 1, **caractérisée en ce que** l'ajustement de l'équilibre des ions est effectué sur de l'eau réduite par électrolyse.

7. Solution saline physiologique artificielle selon la revendication 1, **caractérisée en ce que** le potentiel d'oxydo-réduction est de -800 mV à +200 mV.

8. Méthode de fabrication d'une solution saline physiologique artificielle selon la revendication 1, **caractérisée par** l'ajustement de l'eau réduite par électrolyse par dilution ou condensation et/ou addition d'au moins l'un parmi le groupe constitué d'agent tampon, d'acide chlorhydrique et d'hydroxyde de sodium de sorte que la valeur de réaction d'hydrogène actif qui est mesurée conformément à la méthode de DBNBS en utilisant du 3,5-dibromo-4-nitrosobenzènesulfonate de sodium (DBNBS) soit de 0,01 à 1, le pH soit de 6,0 à 7,9 et la pression osmotique soit de 260 mOsm/L à 320 mOsm/L.

9. Méthode de fabrication d'une solution saline physiologique artificielle selon la revendication 8, **caractérisée en ce qu'**elle comprend en outre l'étape consistant à ajuster l'équilibre des ions dans de l'eau réduite par électrolyse en ajoutant du chlorure de sodium et/ou du chlorure de potassium.

10. Solution saline physiologique artificielle, **caractérisée en ce que** la valeur de réaction d'hydrogène actif qui est mesurée conformément à la méthode de DBNBS en utilisant du 3,5-dibromo-4-nitrosobenzènesulfonate de sodium (DBNBS) est de 0,01 à 1, le pH est de 6,0 à 7,9 et la pression osmotique est de 260 mOsm/L à 320 mOsm/L, destinée à être utilisée comme une solution de nettoyage d'organe.

11. Utilisation d'une solution saline physiologique artificielle, **caractérisée en ce que** la valeur de réaction d'hydrogène actif qui est mesurée conformément à la méthode de DBNBS en utilisant du 3,5-dibromo-4-nitrosobenzènesulfonate de sodium (DBNBS) est de 0,01 à 1, le pH est de 6,0 à 7,9 et la pression osmotique est de 260 mOsm/L à 320 mOsm/L, pour la fabrication d'une solution de nettoyage d'organe.
